# EUROPEAN PATENT SPECIFICATION

(11) **EP 2 457 540 B1**
(45) Date of publication and mention of the grant of the patent: **03.09.2014**
(21) Application number: 12152927.5
(22) Date of filing: 29.06.2010
(51) Int. Cl.: A61F 2/34, A61F 2/38, A61F 2/44, A61B 17/68

(54) **Screw thread placement in a porous medical device**
Schraubengewindeplatzierung in einer porösen medizinischen Vorrichtung
Placement du filetage d'une vis dans un dispositif médical poreux

(30) Priority: 30.06.2009 US 221614 P
(43) Date of publication of application: 30.05.2012
(62) Divisional of application: 10731670.5
(73) Proprietor: Zimmer, Inc., Warsaw, IN 46580 (US)
(72) Inventor: Mohammed, Mobarakh, Bronx, NY New York 10463-2951 (US)
(74) Representative: Mays, Julie

(56) References cited:
- WO-A1-2007/016796
- US-A1- 2008 004 626

## Description

### BACKGROUND

### 1. Field of the Invention.

The present invention relates to orthopedic implants. More particularly, the present invention relates to placement of internal screw threads in porous orthopedic implants.

### 2. Description of the Related Art.

Orthopedic implants include porous bone contacting surfaces to encourage bone ingrowth. Bone ingrowth promotes increased fixation of the orthopedic implant to adjacent bone tissue. However, compared to a solid structure, the high porosity causes the orthopedic implant to have a reduced surface area available for engaging adjacent orthopedic components. The high porosity also impacts the ability to machine the orthopedic implant at close tolerance.

Document WO 2007016796 A1 discloses an implant according to the preamble of claim 1.

### SUMMARY

The present invention provides a porous orthopedic implant according to claim 1, such as an orthopedic anchor. In an exemplary implant not falling within the scope of the claims, an internal thread is formed in the orthopedic implant by bonding a preformed, internally threaded component to the orthopedic implant. In another exemplary implant not falling within the scope of the claims, an internal thread is formed in the orthopedic implant by bonding a solid insert to the orthopedic implant and then forming the thread into that solid insert. The internal thread is formed in the orthopedic implant according to the invention by forming a surface coating on the orthopedic implant and then forming the thread into that surface coating.

According to an embodiment of the present invention, a porous orthopedic implant is provided to receive a threaded fastener. The porous orthopedic implant includes a porous body having an outer surface for contacting a patient's bone, the porous body including an internal bore defined by an internal wall of the porous body, and an insert located within the internal bore of the porous body, the insert coupled to the internal wall of the porous body to resist rotation and axial translation of the insert relative to the porous body, the insert defining a thread that is configured to receive the threaded fastener.

According to another exemplary implant not falling within the scope of the claims, a porous orthopedic implant is provided to receive a threaded fastener. The porous orthopedic implant includes a first, porous component that defines an outer surface of the porous orthopedic implant for contacting a patient's bone, and a second component that is less porous than the first component, the second component coupled to the first component to resist rotation and axial translation of the second component relative to the first component, the second component defining an internal thread of the porous orthopedic implant for receiving the threaded fastener.

Also described herein is a method for manufacturing a porous orthopedic implant that is configured to receive a threaded fastener. The method includes the steps of providing a porous body having an outer surface for contacting a patient's bone, the porous body including an internal bore defined by an internal wall of the porous body, and coupling an insert to the internal wall of the porous body to resist rotation and axial translation of the insert relative to the porous body, the insert defining a thread that cooperates with the threaded fastener.

### BRIEF DESCRIPTION OF THE DRAWINGS

The above-mentioned and other features and advantages of this invention, and the manner of attaining them, will become more apparent and the invention itself will be better understood by reference to the following description of exemplary implants taken in conjunction with the accompanying drawings, wherein:
Figure 1 is a perspective view of a patient's femur having an exemplary orthopedic implant in the form of a porous anchor implanted therein, the anchor shown being coupled to a screw and a washer;
Figure 2 is a cross-sectional view of the anchor of Figure 1, showing a wire thread insert bonded to the anchor not falling within the scope of the claims;
Figure 3 is another cross-sectional view of the anchor of Figure 1, showing a wire thread insert bonded to the anchor with an intermediate polymer layer;
Figure 4 is another cross-sectional view of the anchor of Figure 1, showing a wire thread insert bonded to the anchor with an intermediate sintered metal powder layer;
Figure 5 is yet another cross-sectional view of the anchor of Figure 1, showing a solid insert bonded to the anchor;
Figure 5A is a view similar to Figure 5, showing an internal thread formed into the solid insert of Figure 5;
Figure 6 is yet another cross-sectional view of the anchor of Figure 1, showing a surface coating on the anchor and an internal thread formed into the surface coating; and
Figure 7 is a perspective view of the anchor of Figure 1 coupled to an acetabular shell and showing a wire thread insert bonded to the anchor not falling within the scope of the claims.

Corresponding reference characters indicate corresponding parts throughout the several views. The exemplifications set out herein illustrate exemplary embodiments of the invention and such exemplifications are not to be construed as limiting the scope of the invention in any manner.

### DETAILED DESCRIPTION

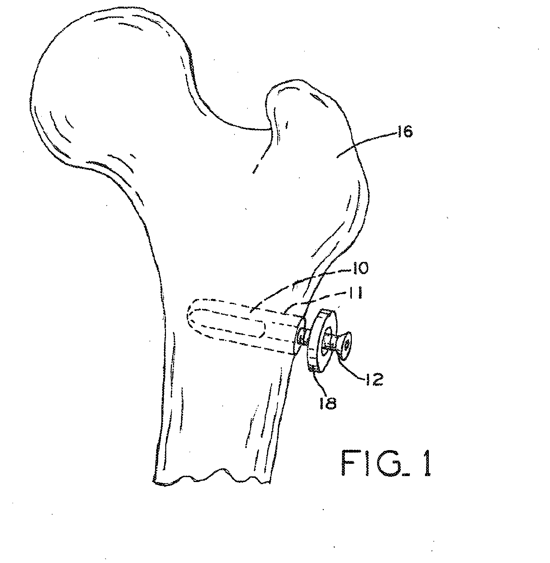

Figure 1 depicts an orthopedic implant in the form of anchor 10. Anchor 10 is shown (in phantom) implanted in a patient's femur 16 such that outer surface 11 of anchor 10 contacts bone and/or soft tissue. Anchor 10 is configured to receive an externally threaded fastener, such as a bolt or screw 12. Coupling a threaded fastener, such as screw 12, to anchor 10 may provide a secure, locked engagement between anchor 10 and another orthopedic implant and/or between anchor 10 and a patient's bone, for example. Although the orthopedic implant is described and depicted herein as anchor 10, the orthopedic implant of the present disclosure may be any suitable implant configured to receive an externally threaded fastener, including, for example, a tibial component (such as a tibial augment component), a femoral component, an acetabular component, or a spinal fusion component (such as a posterior lumbar interbody fusion implant).

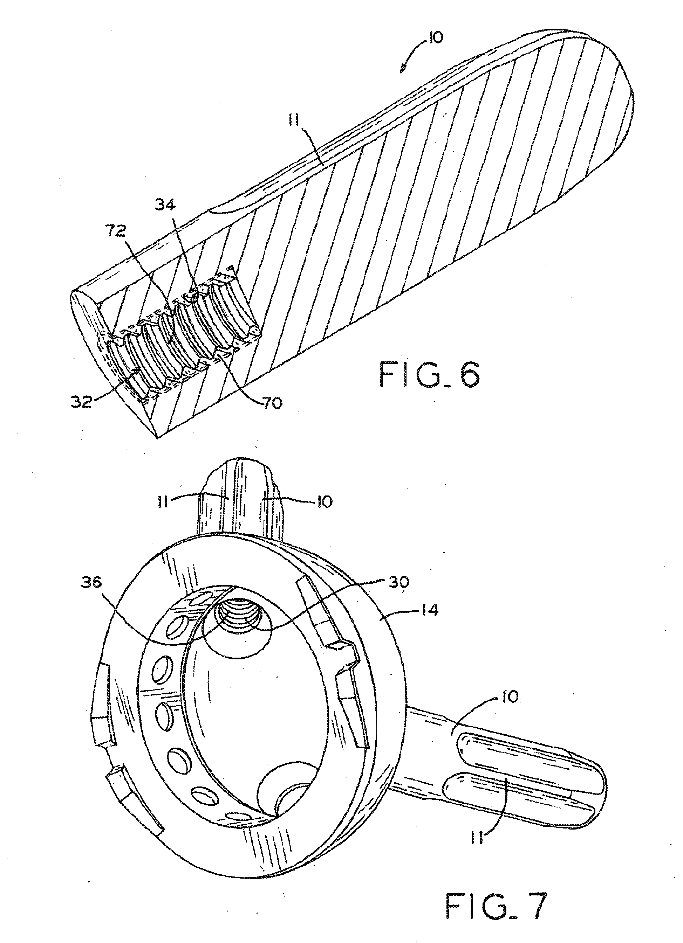

Anchor 10 may be used in a variety of applications. For example, as shown in Figure 1, anchor 10 is implanted in a patient's femur 16 to receive screw 12 and washer 18 for clamping ligaments, tendons, muscles, or other soft tissue structures against femur 16. As another example, and as shown in Figure 7, anchors 10 are coupled to acetabular shell 14 to secure acetabular shell 14 to a patient's pelvis (not shown). An exemplary anchor and acetabular shell assembly is described in U.S. Patent Publication No. 2008/0046091 to Weiss et al., entitled "IMPLANT ANCHORING DEVICE," filed March 19, 2007, and assigned to the assignee of the present application.

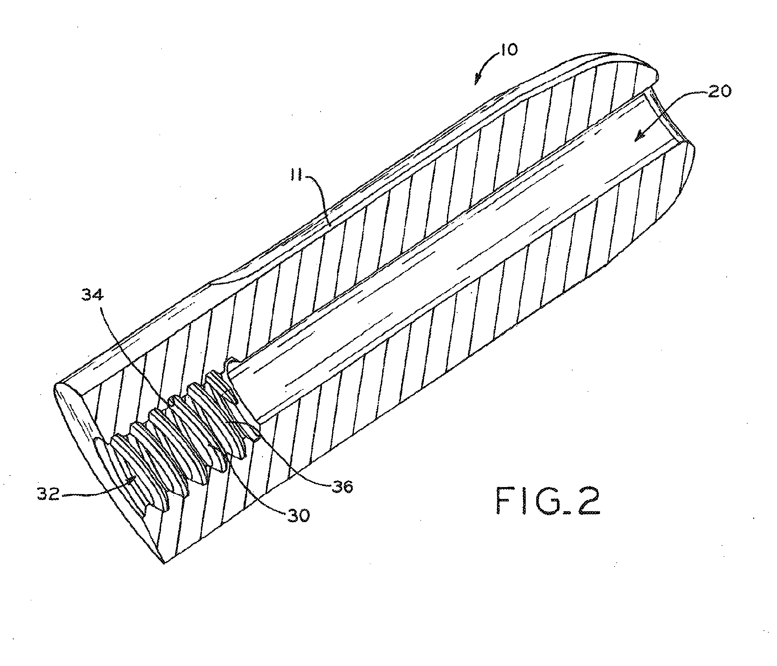

Referring next to Figure 2, anchor 10 may define a hollow chamber 20 that extends entirely through anchor 10 such that anchor 10 is cannulated and capable of receiving a guide wire or another insertion tool, for example. It is also within the scope of the present invention that anchor 10 may be an expandable device. For example, anchor 10 may include radially displaceable fingers (not shown) such that insertion of screw 12 (Figure 1) into anchor 10 causes anchor 10 to expand.

Anchor 10 may be constructed of a highly porous, open-cell material to encourage bone growth into anchor 10. As used herein, an "open-cell material" is a material containing pores that are connected to each other and form an interconnected network. Anchor 10 may have a porosity as low as 55, 60, or 65 percent and as high as 80, 85, or 90 percent or more.

An example of such a material is produced using Trabecular Metal™ technology generally available from Zimmer, Inc., of Warsaw, Indiana. Trabecular Metal™ is a trademark of Zimmer Technology, Inc. Such a material may be formed from a reticulated vitreous carbon foam substrate which is infiltrated and coated with a biocompatible metal, such as tantalum, by a chemical vapor deposition ("CVD") process in the manner disclosed in detail in U.S. Patent No. 5,282,861 to Kaplan, entitled "OPEN CELL TANTALUM STRUCTURES FOR CANCELLOUS BONE IMPLANTS AND CELL AND TISSUE RECEPTORS," filed February 1, 1994. In addition to tantalum, other metals such as niobium, or alloys of tantalum and niobium with one another or with other metals may also be used.

Generally, the porous tantalum structure includes a large plurality of ligaments defining the open cells, or open spaces, therebetween, with each ligament generally including a carbon core covered by a thin film of metal such as tantalum, for example. The open spaces between the ligaments form a matrix of continuous channels having no dead ends, such that growth of cancellous bone through the porous tantalum structure is uninhibited. The porous tantalum may have a porosity as low as 55, 60, or 65 percent and as high as 80, 85, or 90 percent or more. Thus, porous tantalum is a lightweight, strong porous structure which is substantially uniform and consistent in composition, and closely resembles the structure of natural cancellous bone, thereby providing a matrix into which cancellous bone may grow to provide fixation of anchor 10 to the patient's bone.

The porous tantalum structure may be made in a variety of densities to selectively tailor the structure for particular applications. In particular, and as discussed in the above-incorporated U.S. Patent No. 5,282,861, the porous tantalum may be fabricated to virtually any desired porosity and pore size, and can thus be matched with the surrounding natural bone to provide an improved matrix for bone ingrowth and mineralization.

As discussed above with respect to Figure 1, anchor 10 is configured to receive an externally threaded fastener, such as screw 12. Although the high porosity of anchor 10 promotes fixation of anchor 10 to the patient's bone via bone ingrowth, the high porosity of anchor 10 reduces the surface area of anchor 10 available to contact and engage screw 12. Therefore, the highly porous anchor 10 may have a low surface area that does not sufficiently contact screw 12 in a tight, friction fit engagement. The high porosity of anchor 10 also impacts the ability to machine anchor 10 at close tolerance. Therefore, it may be difficult to machine an internal thread directly into anchor 10 with adequate accuracy and consistency.

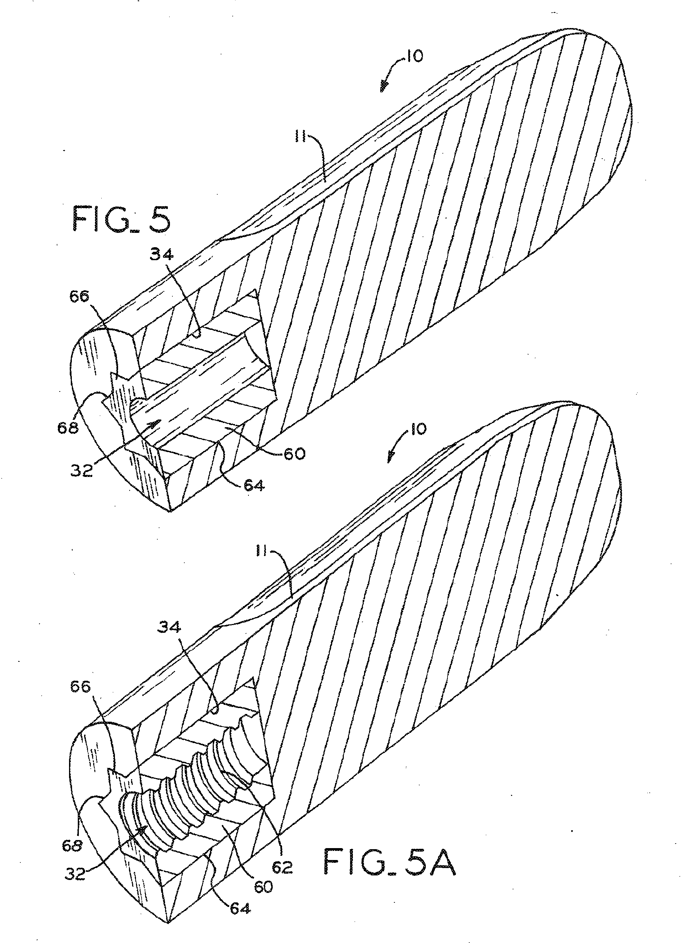

The present disclosure describes various methods for manufacturing an internal thread in anchor 10, notwithstanding the high porosity of anchor 10. In an exemplary implant not falling within the scope of the claims, and as shown in Figures 2-4, an internal thread is formed in anchor 10 by bonding a pre-formed, internally threaded component, such as a metallic wire thread insert 30, to anchor 104. Suitable wire thread inserts 30 include Spiralock^{™} wire thread inserts and Helicoil^{™} wire thread inserts, both of which are generally available from Emhart Teknologies of Shelton, Connecticut. As shown in Figures 1 and 2, wire thread insert 30 is a tightly wound, helical coil that forms internal thread 36, which is configured to cooperate with a corresponding threaded fastener, such as screw 12. In another embodiment, and as shown in Figures 5-6, an internal thread is formed in anchor 10 by bonding a solid plug 60 to anchor 10 or forming a surface coating 70 on anchor 10, and then machining thread 62, 72, into that plug 60 or surface coating 70.

According to an exemplary implant not falling within the scope of the claims, wire thread insert 30 is bonded to anchor 10 via diffusion bonding, as shown in Figure 2. The diffusion bonding process may be performed according to the method disclosed in U.S. Patent Publication No. 2008/0195222 to Rauguth et al., entitled "DIRECT APPLICATION OF PRESSURE FOR BONDING POROUS COATINGS TO SUBSTRATE MATERIALS USED IN ORTHOPAEDIC IMPLANTS," filed March 2, 2007.

First, anchor 10 is prepared to receive wire thread insert 30. Preparing anchor 10 to receive wire thread insert 30 may involve molding anchor 10 to include a suitably sized bore 32 or drilling bore 32 into anchor 10 post-manufacturing. Also, preparing anchor 10 to receive wire thread insert 30 may involve shaping or tapping internal wall 34 of anchor 10 surrounding bore 32 to engage wire thread insert 30. Wire thread insert 30 is then inserted into bore 32 of anchor 10. During subsequent insertion of screw 12 (Figure 1), wire thread insert 30 may expand outwardly against internal wall 34 of anchor 10 to provide a tight, friction fit engagement between screw 12, wire thread insert 30, and anchor 10, which reduces the need to machine internal wall 34 of anchor 10 at close tolerance.

Next, wire thread insert 30 is fused to anchor 10 via diffusion bonding. For example, wire thread insert 30 may be held against internal wall 34 of anchor 10 under an applied pressure while the components are heated to an elevated temperature for a time ranging from a few minutes to a few hours. The diffusion bonding process may be performed in a protective, inert atmosphere or under a vacuum, for example. The elevated temperature may be less than the melting point of both components. Advantageously, the diffusion bonding process may eliminate gaps between the components to fuse the highly porous anchor 10 and wire thread insert 30 together, even if internal wall 34 of anchor 10 is not initially machined at close tolerance.

According to another exemplary implant not according to the present invention, wire thread insert 30 is bonded to anchor 10 via CVD processing. The CVD process may be performed according to the method disclosed in the above U.S. Patent No. 5,282,861.

First, anchor 10 is formed to less than its final density. For example, anchor 10 may be formed to less than its final density by heating a suitably shaped carbon foam substrate in a hot wall furnace in the presence of tantalum chloride gas and hydrogen gas to deposit a first amount of tantalum on and within the carbon foam substrate. Wire thread insert 30 is then inserted into bore 32 of the partially coated anchor 10.

Next, with wire thread insert 30 positioned in bore 32 of the partially coated anchor 10, anchor 10 is formed to its final density. For example, anchor 10 may be formed to its final density by returning the partially coated anchor 10 and wire thread insert 30 to the hot wall furnace for further heating in the presence of tantalum chloride gas and hydrogen gas to deposit a second amount of tantalum on the partially coated anchor 10. Advantageously, in addition to forming anchor 10 to its final, implantable density, this subsequent CVD step deposits metal between internal wall 34 of anchor 10 and wire thread insert 30 to fill in gaps between the components and/or interdigitate with the components to fuse the highly porous anchor 10 and wire thread insert 30 together, even if internal wall 34 of anchor 10 is not initially machined at close tolerance. Wire thread insert 30 may be shielded, as necessary, to avoid unwanted deposition of tantalum onto wire thread insert 30 itself.

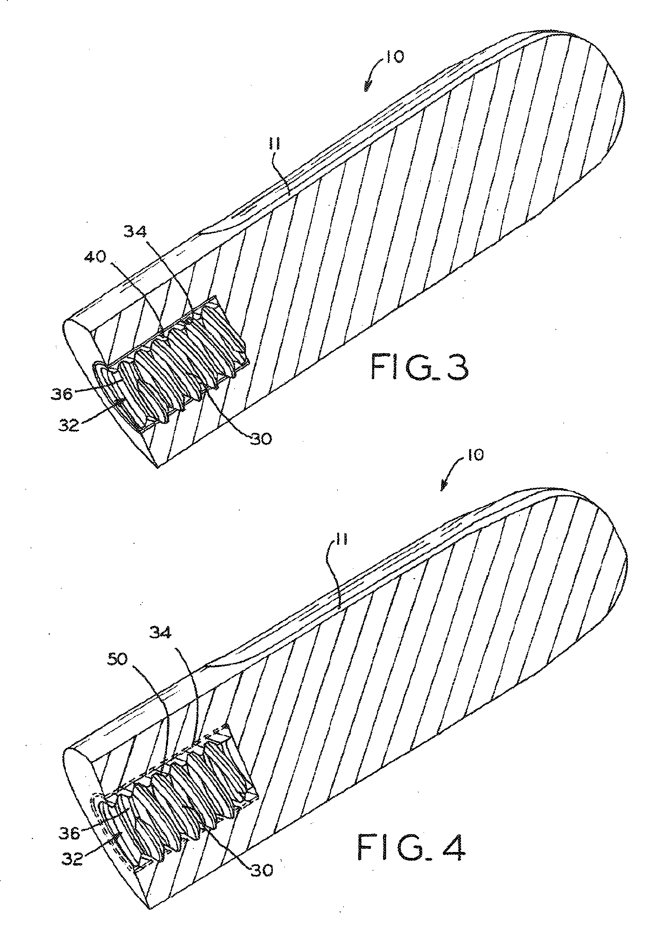

According to another exemplary implant not according to the present invention, and as shown in Figure 3, wire thread insert 30 is bonded to anchor 10 with an intermediate polymer layer 40. The polymer bonding process may be performed according to the method disclosed in U.S. Patent Publication No. 2005/0184134 to Charlebois et al., entitled "METHOD FOR ATTACHING A POROUS METAL LAYER TO A METAL SUBSTRATE," filed April 18, 2005.

First, anchor 10 is prepared to receive both wire thread insert 30 and polymer layer 40. Preparing anchor 10 to receive wire thread insert 30 and polymer layer 40 may involve molding anchor 10 to include a suitably sized bore 32 or drilling bore 32 into anchor 10 post-manufacturing. Bore 32 of anchor 10 may be sized such that internal wall 34 of anchor 10 at least partially contacts wire thread insert 30, such as along the widest portions of wire thread insert 30. Alternatively, bore 32 of anchor 10 may be sized such that internal wall 34 of anchor 10 avoids contact with wire thread insert 30, with intermediate polymer layer 40 separating internal wall 34 of anchor 10 from even the widest portions of wire thread insert 30.

Next, polymer layer 40 is compression molded, injection molded, or otherwise applied to internal wall 34 of anchor 10. For example, polymer layer 40 may be compression molded or injection molded into bore 32 of anchor 10 to substantially fill bore 32. According to an exemplary embodiment, polymer layer 40 is applied to at least partially interdigitate into the highly porous anchor 10. The polymer material may include a biocompatible polymer, such as polyethylene, poly ether ether ketone (PEEK), polyaryl ether ketone (PEAK), ultra polyaryl ether ketone (Ultra PEAK), or another suitable biocompatible polymer.

Then, after polymer layer 40 has sufficiently hardened, polymer layer 40 is machined or tapped to receive wire thread insert 30. Allowing polymer layer 40 to harden before subjecting polymer layer 40 to machining or tapping may encourage polymer layer 40 to interdigitate into and form a strong connection with the highly porous anchor 10, but it is also within the scope of the present disclosure that a still-soft polymer layer 40 may be shaped to receive wire thread insert 30. This machining or tapping step may be performed using a tool that is provided by the manufacturer of the particular wire thread insert 30. If polymer layer 40 is initially applied to substantially or entirely fill bore 32 of anchor 10, such as when injecting a polymer material into bore 32 of anchor 10, a substantial portion of polymer layer 40 may be removed during this machining step.

Finally, wire thread insert 30 is inserted into bore 32 of anchor 10 to contact the tapped polymer layer 40. Alternatively, it is within the scope of the present invention that wire thread insert 30 may be press-fit into polymer layer 40 while polymer layer 40 is somewhat softened, such as by the application of heat. Advantageously, the hardened intermediate polymer layer 40 may fill in gaps and/or interdigitate with the highly porous anchor 10 and form a substantially solid surface for engagement with wire thread insert 30, even if internal wall 34 of anchor 10 is not initially machined at close tolerance.

According to another exemplary embodiment of the present disclosure, and as shown in Figure 4, wire thread insert 30 is bonded to anchor 10 with an intermediate sintered metal powder layer 50. The sintering process may be performed according to the method disclosed in the above-incorporated U.S. Patent Publication No. 2005/0184134.

First, anchor 10 is prepared to receive both wire thread insert 30 and metal powder layer 50. Preparing anchor 10 to receive wire thread insert 30 and metal powder layer 50 may involve molding anchor 10 to include a suitably sized bore 32 or drilling bore 32 into anchor 10 post-manufacturing. Bore 32 of anchor 10 may be sized such that internal wall 34 of anchor 10 at least partially contacts wire thread insert 30, such as the widest portions of wire thread insert 30. Alternatively, bore 32 of anchor 10 may be sized such that internal wall 34 of anchor 10 avoids contact with wire thread insert 30, with intermediate metal powder layer 50 separating internal wall 34 of anchor 10 from even the widest portions of wire thread insert 30.

Next, metal powder layer 50 is sprayed, painted, injected, or otherwise applied to internal wall 34 of anchor 10. For example, metal powder layer 50 may be injected into bore 32 of anchor 10 to substantially fill bore 32. According to an exemplary embodiment, metal powder layer 50 is applied to at least partially interdigitate into the highly porous anchor 10. It is also within the scope of the present invention that metal powder layer 50 may be applied to wire thread insert 30 instead of or in addition to anchor 10. Suitable biocompatible metal powders include, for example, stainless steel, cobalt-chrome alloy, hafnium, manganese, niobium, palladium, titanium-6, aluminum-4, vanadium alloy, aluminum-7, titanium-nickel alloy, zirconium, zirconium alloys, Ti-6Al-4V, Ti-6Al-7Nb, commercially pure titanium, titanium alloys, and cobalt-chromium-molybdenum. The metal powder may be accompanied by an organic binder that is configured to hold the metal powder in place initially and decompose upon heating. Suitable organic binders include, for example, gelatin, glycerin, polyvinyl alcohol (PVA), or a combination of the same.

Next, the assembly is heated to sinter the metal powder particles to each other and to internal wall 34 of anchor 10. The sintering process may be performed in a protective, inert atmosphere or under a vacuum and for a time ranging from a few minutes to a few hours, for example.

Then, after metal powder layer 50 has sufficiently hardened, metal powder layer 50 is machined or tapped to receive wire thread insert 30. Allowing metal powder layer 50 to harden before subjecting metal powder layer 50 to machining or tapping may encourage metal powder layer 50 to interdigitate into and form a strong connection with the highly porous anchor 10. This machining or tapping step may be performed using a tool that is provided by the manufacturer of the particular wire thread insert 30. If metal powder layer 50 is initially applied to substantially or entirely fill bore 32 of anchor 10, such as when injecting metal powder layer 50 into bore 32 of anchor 10, a substantial portion of metal powder layer 50 may be removed during this machining step.

Finally, wire thread insert 30 is inserted into bore 32 of anchor 10 to contact the tapped metal powder layer 50. Alternatively, it is within the scope of the present invention that wire thread insert 30 may be press-fit into metal powder layer 50 and the entire assembly heated to sinter the metal powder particles to each other, to internal wall 34 of anchor 10, and to wire thread insert 30. In this embodiment, wire thread insert 30 may be held against metal powder layer 50 and internal wall 34 of anchor 10 under an applied pressure while the components are heated to an elevated temperature for a time ranging from a few minutes to a few hours. The sintering process may be performed in a protective, inert atmosphere or under a vacuum, for example. Advantageously, upon heating, the intermediate metal powder layer 50 may fill in gaps and/or interdigitate with the highly porous anchor 10 and form a substantially solid surface for engagement with wire thread insert 30, even if internal wall 34 of anchor 10 is not initially machined at close tolerance.

According to yet another exemplary implant not according to the present invention, and as shown in Figures 5 and 5A, plug 60 is bonded to anchor 10, and then internal thread 62 is machined into plug 60. Plug 60 may be constructed of a biocompatible metal, a rigid polymer such as polyethylene, or another suitable material having a porosity less than that of anchor 10 and may be provided in an already-solid form. Unlike the embodiments described above, in which anchor 10 receives wire thread insert 30 which in turn engages screw 12 (Figure 1), the machined internal thread 62 itself may be configured to engage screw 12.

First, anchor 10 is prepared to receive plug 60. Preparing anchor 10 to receive plug 60 may involve molding anchor 10 to include a suitably sized bore 32 or drilling bore 32 into anchor 10 post-manufacturing. Also, preparing anchor 10 to receive plug 60 may involve shaping internal wall 34 of anchor 10 surrounding bore 32 to engage external surface 64 of plug 60. For example, as shown in Figure 5, external surface 64 of plug 60 includes radially spaced protrusions 66, and internal wall 34 of anchor includes corresponding indentations 68, to increase the surface area of contact between the components for improved bonding.

Next, plug 60 is inserted into bore 32 of anchor and bonded to internal wall 34 that surrounds bore 32 of anchor 10. Plug 60 may be initially press-fit into bore 32. However, because bore 32 of porous anchor 10 may not be formed at close tolerance, additional steps may be necessary to securely bond plug 60 to anchor 10. Suitable methods for bonding plug 60 to anchor 10 are described above with respect to wire thread insert 30. For example, plug 60 may be fused to anchor 10 via diffusion bonding, CVD processing, an intermediate polymer layer, an intermediate sintered metal layer, or another suitable process.

Then, internal thread 62 is machined, tapped, or otherwise formed into plug 60, as shown in Figure 5A. The machined internal thread 62 of plug 60 is configured to cooperate with a corresponding threaded fastener, such as screw 12 (Figure 1).

According to the present invention, and as shown in Figure 6, internal surface coating 70 is applied to anchor 10, and then internal thread 72 is machined into internal surface coating 70. Surface coating 70 may be formed of a biocompatible metal, a polymer such as polyethylene, or another suitable material to form a surface layer having a porosity less than that of anchor 10 itself. Unlike the embodiments described above, in which anchor 10 receives wire thread insert 30 which in turn engages screw 12 (Figure 1), the machined internal thread 72 itself may be configured to engage screw 12.

First, anchor 10 is prepared to receive surface coating 70. Preparing anchor 10 to receive surface coating 70 may involve molding anchor 10 to include a suitably sized bore 32 or drilling bore 32 into anchor 10 post-manufacturing.

Next, surface coating 70 is sprayed, painted, injected, compressed, or otherwise applied onto internal wall 34 of anchor 10. Like the embodiments described above for receiving wire thread insert 30, surface coating 70 may be injected into bore 32 of anchor 10 to substantially fill bore 32. An exemplary surface coating 70 includes a metal powder, and optionally an organic binder, as described above. When using a metal powder, the coating step may involve injecting a metal powder and a binder into bore 32 of anchor 10 to substantially or entirely fill bore 32 and interdigitate into the highly porous anchor 10. The coating step may also involve heating the components to an elevated temperature to sinter the metal powder particles to each other and to internal wall 34 of anchor 10. The sintering process may be performed in a protective, inert atmosphere or under a vacuum, for example. Another exemplary surface coating 70 includes a rigid polymer such as polyethylene. When using a polymer material, the coating step may involve compression molding or injection molding a soft or fluid polymer into bore 32 of anchor 10 to substantially or entirely fill bore 32 and interdigitate into the highly porous anchor 10.

Then, after surface coating 70 has sufficiently hardened, internal thread 72 is machined, tapped, or otherwise formed into surface coating 70, as shown in Figure 6. Allowing surface coating 70 to harden before subjecting surface coating 70 to machining or tapping may encourage surface coating 70 to interdigitate into and form a strong connection with the highly porous anchor 10. If surface coating 70 is initially applied to substantially or entirely fill bore 32 of anchor 10, such as when injecting a metal powder or a polymer into bore 32 of anchor 10, a substantial portion of surface coating 70 may be removed during this machining step. Internal thread 72 is configured to cooperate with a corresponding threaded fastener, such as screw 12 (Figure 1).

The methods described herein accommodate standard components, such as commercially available screws 12 and corresponding wire thread inserts 30. Current methods require the use of custom-manufactured, internally threaded components that are keyed or specially shaped to resist rotation relative to the porous orthopedic implant. These internally threaded components are not bonded to the porous orthopedic implant, so to resist axial pull-out, the fasteners must extend into an adjacent, non-porous implant.

Additionally, the methods described herein avoid having to machine bore 32 in porous anchor 10 at close tolerance. Current methods require the use of specially shaped bores in the porous orthopedic implant that are sized to receive a similarly shaped threaded component. The wall of the porous orthopedic implant surrounding the specially shaped bore must be machined to frictionally engage the threaded component to resist rotation of the threaded component relative to the porous orthopedic implant. Such methods utilize expensive and time-consuming procedures to shape the porous orthopedic implant, such as electro discharge machining (EDM).

While this invention has been described as having preferred designs, the present invention can be further modified within the scope of this disclosure. This application is therefore intended to cover any variations, uses, or adaptations of the invention using its general principles. Further, this application is intended to cover such departures from the present disclosure as come within known or customary practice in the art to which this invention pertains and which fall within the limits of the appended claims.

## Claims

1. A porous orthopedic implant (10) that is configured to receive a threaded fastener (12), the porous orthopedic implant comprising:
a porous body having an outer surface for contacting a patient's bone, the porous body including an internal bore defined by an internal wall of the porous body; and
an insert (70) located within the internal bore of the porous body, the insert coupled to the internal wall of the porous body to resist rotation and axial translation of the insert relative to the porous body, the insert defining a thread (72) that is configured to receive the threaded fastener, **characterised in that** the insert is a coating (70) that is machined to form the thread (72).

2. The porous orthopedic implant of claim 1, wherein the porous body has a porosity in the range of 55-90 percent.

3. The porous orthopedic implant of any of claims 1-2, wherein the insert is formed of a biocompatible metal.

4. The porous orthopedic implant of any of claims 1-3, wherein the coating is hardened.

5. The porous orthopedic implant of any of claims 1-4, wherein the insert interdigitates into pores in the porous body.

6. The porous orthopedic implant of any of claims 1-5, wherein the coating results from a coating material sprayed, painted, injected, or compressed onto the internal wall.

7. The porous orthopedic implant of any of claims 1-6, wherein the insert has a porosity less than that of said porous body.

8. The porous orthopedic implant of any of claims 1-7, wherein the porous body is at least one of an anchor, a tibial component, a femoral component, an acetabular component, and a spinal fusion component.

## Patentansprüche

1. Poröses orthopädisches Implantat (10), das zur Aufnahme eines mit Gewinde versehenen Befestigungselements (12) ausgestaltet ist, wobei das poröse orthopädische Implantat umfasst:
einen porösen Körper mit einer Außenfläche zum In-Kontakt-Bringen mit einem Knochen eines Patienten, wobei der poröse Körper eine durch eine Innenwand des porösen Körpers definierte innere Bohrung umfasst; und
einen in der inneren Bohrung des porösen Körpers befindlichen Einsatz (70), wobei der Einsatz so mit der Innenwand des porösen Körpers verbunden ist, dass sich der Einsatz bezogen auf den porösen Körper weder dreht noch axial verschiebt, wobei der Einsatz ein zur Aufnahme des mit Gewinde versehenen Befestigungselements ausgestaltetes Gewinde (72) definiert,
**dadurch gekennzeichnet, dass** der Einsatz eine Beschichtung (70) ist, die so bearbeitet ist, dass sie das Gewinde (72) ausbildet.

2. Poröses orthopädisches Implantat nach Anspruch 1, wobei der poröse Körper eine Porosität im Bereich von 55-90 Prozent aufweist.

3. Poröses orthopädisches Implantat nach einem der Ansprüche 1-2, wobei der Einsatz aus einem biokompatiblen Metall ausgebildet ist.

4. Poröses orthopädisches Implantat nach einem der Ansprüche 1-3, wobei die Beschichtung gehärtet ist.

5. Poröses orthopädisches Implantat nach einem der Ansprüche 1-4, wobei der Einsatz in Poren des porösen Körpers eingreift.

6. Poröses orthopädisches Implantat nach einem der Ansprüche 1-5, wobei die Beschichtung durch Sprühen, Streichen, Spritzen oder Pressen eines Beschichtungsmaterials auf die Innenwand ausgebildet wird.

7. Poröses orthopädisches Implantat nach einem der Ansprüche 1-6, wobei der Einsatz eine Porosität aufweist, die geringer ist als die des porösen Körpers.

8. Poröses orthopädisches Implantat nach einem der Ansprüche 1-7, wobei der poröse Körper wenigstens eines der Folgenden ist: ein Anker, eine Schienbeinkomponente, eine Oberschenkelkomponente, eine Komponente des Acetabulums und eine Spondylodesekomponente.

## Revendications

1. Implant orthopédique poreux (10) qui est configuré pour recevoir une attache filetée (12), l'implant orthopédique poreux comprenant :
un corps poreux ayant une surface externe pour venir en contact avec un os d'un patient, le corps poreux comprenant un alésage interne défini par une paroi interne du corps poreux ; et
un insert (70) situé au sein de l'alésage interne du corps poreux, l'insert étant couplé à la paroi interne du corps poreux pour résister à une rotation et à une translation axiale de l'insert par rapport au corps poreux, l'insert définissant un filet (72) qui est configuré pour recevoir l'attache filetée, **caractérisé en ce que** l'insert est un revêtement (70) qui est usiné pour former le filet (72).

2. Implant orthopédique poreux selon la revendication 1, dans lequel le corps poreux a une porosité dans la plage de 55 à 90 pour cent.

3. Implant orthopédique poreux selon l'une quelconque des revendications 1 à 2, dans lequel l'insert est formé d'un métal biocompatible.

4. Implant orthopédique poreux selon l'une quelconque des revendications 1 à 3, dans lequel le revêtement est durci.

5. Implant orthopédique poreux selon l'une quelconque des revendications 1 à 4, dans lequel l'insert s'interdigite en pores dans le corps poreux.

6. Implant orthopédique poreux selon l'une quelconque des revendications 1 à 5, dans lequel le revêtement résulte d'un matériau de revêtement pulvérisé, peint, injecté ou compressé sur la paroi interne.

7. Implant orthopédique poreux selon l'une quelconque des revendications 1 à 6, dans lequel l'insert a une porosité inférieure à celle dudit corps poreux.

8. Implant orthopédique poreux selon l'une quelconque des revendications 1 à 7, dans lequel le corps poreux est au moins l'un parmi un ancrage, un composant tibial, un composant fémoral, un composant acétabulaire et un composant de spondylodèse.
